(19) Europäisches Patentamt
European Patent Office
Office européen des brevets

(11) Veröffentlichungsnummer: **0 036 840**
**A2**

(12) # EUROPÄISCHE PATENTANMELDUNG

(21) Anmeldenummer: **81810107.3**

(22) Anmeldetag: **19.03.81**

(51) Int. Cl.³: **C 07 C 25/02**, C 07 C 17/12, C 09 B 5/32

(30) Priorität: **25.03.80 CH 2324/80**

(43) Veröffentlichungstag der Anmeldung: **30.09.81 Patentblatt 81/39**

(84) Benannte Vertragsstaaten: **CH DE FR GB LI**

(71) Anmelder: **CIBA-GEIGY AG, Patentabteilung Postfach, CH-4002 Basel (CH)**

(72) Erfinder: **Tzikas, Athanassios, Dr., Muttenzerstrasse 78, CH-4133 Pratteln (CH)**

(54) **Verfahren zur Herstellung von chlorierten Verbindungen.**

(57) Verfahren zur Herstellung von mono- und dichlorierten Bis-(perhalogenmethyl)-benzolen der Formel (I) durch Umsetzung von Bis-(perhalogenmethyl)-benzolen der Formel (II) mit elementarem Chlor in Chlorsulfonsäure als Reaktionsmedium bei Temperaturen von vorzugsweise 0 bis 10° C. Die vorliegende Erfindung ermöglicht in einem Verfahrensschritt in hohen Ausbeuten reine Verbindungen der Formel (I) zu erhalten.

EP 0 036 840 A2

CIBA-GEIGY AG
4002 Basel (Schweiz)

1-12776/=

Verfahren zur Herstellung von chlorierten Verbindungen

Die Erfindung betrifft ein Verfahren zur Herstellung von Verbindungen der allgemeinen Formel

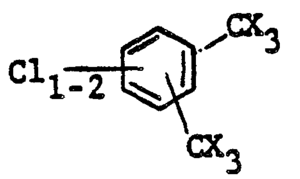

(I) ,

worin X gleiches oder verschiedenes Halogen bedeutet, welches dadurch gekennzeichnet ist, dass man Verbindungen der allgemeinen Formel

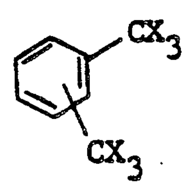

(II) ,

in Chlorsulfonsäure bei Temperaturen von -10 bis +25°C mit elementarem Chlor umsetzt.

Aus der Literatur ist bekannt, Verbindungen der Formel (I) durch Umsetzung von Verbindungen der Formel (II) mit elementarem Chlor bei Temperaturen von 114 bis 185°C (vgl. U.S. Patent 2,394,442; U.S. Patent 2,601,310; J. Org. Chem. 1976, 3580; J. Amer. Chem. Soc. 69, 947) herzustellen, wobei Mischungen mono- und dichlorierter Verbindungen bzw. Isomerengemische isoliert werden, so dass aufwendige Trennoperationen notwendig sind. Umsetzungen von Verbindungen der Formel (II) in Sulfurylchlorid und Dischwefeldichlorid (vgl. Macromolecules 1974, 732) haben einen ungüstigen Reagenzienverbrauch und aufwendige Reinigungsschritte zum Nachteil. Bei der grosstechnischen Herstellung der genannten Verbindungen sind Umsetzungen bei hohen Temperaturen, sowie zusätzliche Reinigungsschritte, die mit geringen Ausbeuten verbunden sind, da kostenaufwendig, unerwünscht.

Der vorliegenden Erfindung lag daher die Aufgabe zugrunde, in einem einzigen Verfahrensschritt, unter Beseitigung der oben aufgeführten Nachteile, reine Verbindungen der Formel (I) in hohen Ausbeuten herzustellen.

Diese Aufgabe wird gemäss vorliegender Erfindung gelöst, indem man Verbindungen der Formel (II) nach oben genanntem Verfahren umsetzt. Ueberraschenderweise werden unter diesen Bedingungen reine Verbindungen der Formel (I) in hohen Ausbeuten erhalten. Das erfindungsgemässe Verfahren ermöglicht somit auf einfache und wirtschaftliche Art und Weise Verbindungen der Formel (I) herzustellen.

In den Verbindungen der Formel (I) können die $CX_3$-Gruppen in o-, m- oder p-Position zueinander stehen. Innerhalb jeder $CX_3$-Gruppe kann gleiches oder verschiedenes Halogen enthalten sein.

Als Ausgangsverbindungen des erfindungsgemässen Verfahrens werden Xylole der Formel (II) eingesetzt, deren Methylgruppen halogeniert sind, vorzugsweise solche, worin X gleiches oder verschiedenes Fluor, Chlor oder Brom ist.

Die Ausgangsverbindungen der Formel (II) sind bekannt, oder können nach bekannten Methoden hergestellt werden.

Als Beispiele seien genannt:

1,2-Bis-(trifluormethyl)-benzol,
1,3-Bis-(trifluormethyl)-benzol,
1-Trichlormethyl-3-trifluormethylbenzol,
1-Tribrommethyl-3-trifluormethylbenzol,
1-Chlordifluormethyl-3-trifluormethylbenzol,
1,3-Bis-(dichlorfluormethyl)-benzol,
1,3-Bis-(trichlormethyl)-benzol,
1-Tribrommethyl-3-trichlormethylbenzol,
1,3-Bis-(tribrommethyl)-benzol,
1,4-Bis-(trifluormethyl)-benzol,
1-Trichlormethyl-4-trifluormethylbenzol,
1-Tribrommethyl-4-trifluormethylbenzol,
1-Dichlorfluormethyl-4-chlordifluormethylbenzol,
1,4-Bis-(trichlormethyl)-benzol,
1-Tribrommethyl-4-trichlormethylbenzol,
1,4-Bis-(tribrommethyl)-benzol.

Als Ausgangsstoffe werden insbesondere 1,3- oder 1,4-Bis-(perhalogenmethyl)-benzole, vorzugsweise 1,3- oder 1,4-Bis-(perchlormethyl)-benzol und 1,3-Bis-(perfluormethyl)-benzol eingesetzt.

Die besonders wertvolle Verbindung der Formel

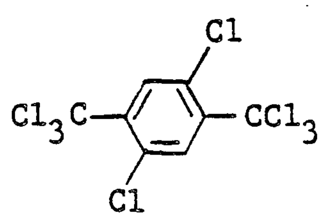

(III),

wird in der Weise hergestellt, dass man 1,4-Bis-(trichlormethyl)-benzol mit Chlor zu der Verbindung der Formel (III) umsetzt.

Die erfindungsgemässe Umsetzung erfolgt in Chlorsulfonsäure als Reaktionsmedium bei Temperaturen von vorzugsweise 0 bis

10°C. Die Umsetzung mit elementarem Chlor wird unter Normaldruck ausgeführt, insbesondere mit katalytischen Mengen Jod.

Die Umsetzung dauert in der Regel 3 bis 5 Stunden und ist von der Geschwindigkeit der Chlorgaszuführung abhängig.

Das Verfahren zur Herstellung mono- oder dichlorierter Produkte wird von den Halogensubstituenten der $CX_3$-Gruppen sowie deren Position im Benzolkern beeinflusst und kann durch die eingesetzten Chlormengen sowie durch die Reaktionstemperatur gesteuert werden.

Zur Aufarbeitung der Verfahrensprodukte trägt man die Suspension auf Eis aus, filtriert, wäscht mit kaltem Wasser neutral und trocknet den Rückstand. Man erhält in hoher Ausbeute reine Produkte.

Die nach dem erfindungsgemässen Verfahren erhaltenen Verbindungen stellen wertvolle Zwischenprodukte zur Herstellung von Farbstoffen, Pharma-, Polymer- und Pigmentprodukten dar. Beispielsweise werden bei der Kondensation der Verfahrensprodukte mit 1,2-Diaminoanthrachinonen wertvolle Küpenfarbstoffe erhalten.

In den folgenden Beispielen bedeuten die Teile, sofern nicht anders angegeben, Gewichtsteile und die Prozente Gewichtsprozente; die Temperaturen sind in Celsiusgraden angegeben. Zwischen Gewichts- und Volumenteilen besteht das gleiche Verhältnis wie zwischen Gramm und Kubikzentimeter.

Beispiel 1

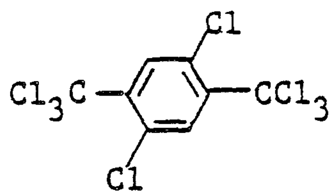

240 Teile Chlorsulfonsäure werden bei Raumtemperatur vorgelegt. Dann werden 62,6 Teile 1,4-Bis-(trichlormethyl)-benzol und 2 Teile Jod zugegeben. Die Suspension wird auf 5° gekühlt und bei 5 bis 10° werden innert 4 Stunden 30 Teile Chlor eingeleitet. Anschliessend wird die Suspension auf Eis ausgetragen und kalt filtriert. Das Nutschgut wird mit eiskaltem Wasser neutral gewaschen.

Der Rückstand wird im Vakuumtrockenschrank bei 60° getrocknet. Es werden 73,7 Teile (96% d.Th.) reines 2,5-Dichlor-1,4-bis-(trichlormethyl)-benzol mit einem Schmelzpunkt von 202 bis 203° erhalten.

Analyse: Chlor berechnet: 74,3%, gefunden: 74,05%.

Beispiel 2

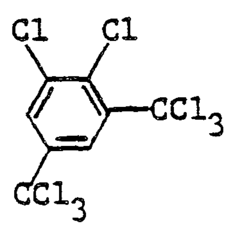

240 Teile Chlorsulfonsäure werden bei Raumtemperatur vorgelegt. Dann werden 62,6 Teile 1,3-Bis-(trichlormethyl)-benzol und 2 Teile Jod zugegeben. Die Suspension wird auf 0° gekühlt und bei 0 bis 5° werden innert ca. 4 Stunden 30 Teile Chlor eingeleitet. Anschliessend wird die Suspension auf Eis ausgetragen und kalt filtriert. Das Nutschgut wird mit eiskaltem Wasser neutral gewaschen.

Der Rückstand wird im Exsikator getrocknet. Es werden 72 Teile 4,5-Dichlor-1,3-bis-(trichlormethyl)-benzol mit einem Schmelzpunkt nach Umkristallisation in Aethanol von 61 bis 62° erhalten.

Analyse: Chlor berechnet: 74,3 %, gefunden: 74,35%.
Die Struktur ist durch Analyse der $^{13}$C-NMR-Daten ermittelt worden.

Beispiel 3

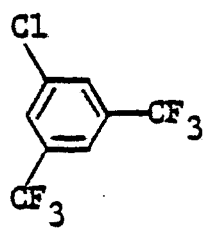

130 Teile Chlorsulfonsäure werden bei Raumtemperatur vorgelegt. Dann werden 42,8 Teile 1,3-Bis-(trifluormethyl)-benzol und 2 Teile Jod zugegeben. Die Suspension wird auf 0° gekühlt und bei 0 bis 5° werden innert 5 Stunden 30 Teile Chlor eingeleitet. Anschliessend wird die Suspension auf Eis ausgetragen und die organische Phase von der wässrigen Phase abgetrennt. Die organische Phase wird mit eiskaltem Wasser neutral gewaschen und über Natriumsulfat calc. getrocknet. Es werden 38 Teile 5-Chlor-1,3-bis-(trifluormethyl)-benzol mit einem Siedepunkt von 111 bis 112° erhalten.

Analyse: Chlor berechnet: 14,28%, gefunden: 14,02%
Fluor berechnet: 45,8%, gefunden: 45,4%.
Die Struktur wurde durch Analyse der $^{13}$C-NMR-Daten ermittelt.

Beispiel 4

19 Teile 2,5-Dichlor-1,4-bis-(trichlormethyl)-benzol und 23,8 Teile 1,2-Diaminoanthrachinon werden in 200 Volumenteilen 98- oder 100%-iger Schwefelsäure bei 130° 4 Stunden verrührt. Nach dem Erkalten wird die Suspension auf Eis/Wasser ausgetragen, filtriert, neutral gewaschen und mit Javell'scher Lauge behandelt. Der so erhaltene Farbstoff der Formel

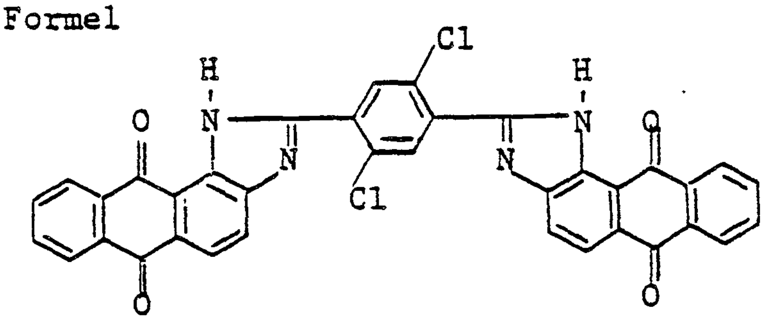

färbt Baumwolle nach der Küpenmethode in grünstichigen gelben Tönen von guten Echtheiten.

Patentansprüche

1. Verfahren zur Herstellung von Verbindungen der allgemeinen Formel

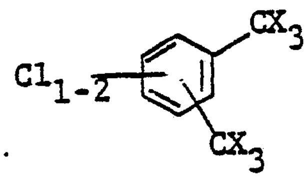

(I) ,

worin X gleiches oder verschiedenes Halogen bedeutet, dadurch gekennzeichnet, dass man Verbindungen der allgemeinen Formel

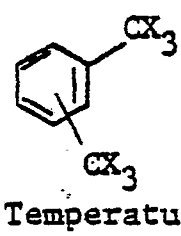

(II) ,

in Chlorsulfonsäure bei Temperaturen von -10 bis +25°C mit elementarem Chlor umsetzt.

2. Verfahren nach Anspruch 1, dadurch gekennzeichnet, dass man Verbindungen der Formel (II), worin X gleiches oder verschiedenes Fluor, Chlor oder Brom ist, als Ausgangsstoffe verwendet.

3. Verfahren nach Ansprüchen 1 und 2, dadurch gekennzeichnet, dass man die Ausgangsverbindungen der Formel(II) bei Temperaturen von 0 bis 10°C mit elementarem Chlor umsetzt.

4. Verfahren nach Ansprüchen 1 bis 3, dadurch gekennzeichnet, dass man die Chlorierung in Gegenwart von katalytischen Mengen Jod ausführt.

5. Verfahren nach Ansprüchen 1 bis 4, dadurch gekennzeichnet, dass man als Ausgangsverbindungen 1,3- oder 1,4-Bis-(perhalogenmethyl)-benzole einsetzt.

6. Verfahren nach Ansprüchen 1, 3 und 4, dadurch gekennzeichnet, dass man als Ausgangsverbindungen 1,3- oder 1,4-Bis-(perchlormethyl)-benzol einsetzt.

7. Verfahren nach Ansprüchen 1, 3 und 4, dadurch gekennzeichnet, dass man als Ausgangsverbindung 1,3-Bis-(perfluormethyl)-benzol einsetzt.

8. Verfahren nach Anspruch 1, zur Herstellung der Verbindung der Formel

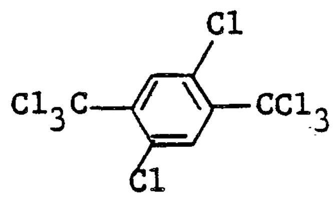

(III) ,

dadurch gekennzeichnet, dass man 1,4-Bis-(trichlormethyl)-benzol mit Chlor zu der Verbindung der Formel (III) umsetzt.

9. Die nach dem Verfahren gemäss den Ansprüchen 1 bis 8 erhaltenen Verbindungen der Formel (I).

10. Verfahren gemäss den gegebenen Ausführungsbeispielen.

11. Die nach dem Verfahren gemäss den gegebenen Ausführungsbeispielen erhaltenen Verbindungen der Formel (I).

12. Verwendung der nach dem Verfahren gemäss den Ansprüchen 1 bis 8 und 10 erhaltenen Verbindungen der Formel (I) zur Herstellung von Küpenfarbstoffen.